# EUROPEAN PATENT SPECIFICATION

(11) **EP 3 329 854 B1**
(45) Date of publication and mention of the grant of the patent: **11.10.2023**
(21) Application number: 16829606.9
(22) Date of filing: 08.04.2016
(51) Int. Cl.: A61B 8/00, A61B 8/08

(54) **THREE-DIMENSIONAL IMAGING ULTRASONIC SCANNING METHOD**
DREIDIMENSIONALES BILDGEBENDES ULTRASCHALLABTASTVERFAHREN
PROCÉDÉ DE BALAYAGE ULTRASONORE D'IMAGERIE TRIDIMENSIONNELLE

(30) Priority: 28.07.2015 CN 201510452165
(43) Date of publication of application: 06.06.2018
(73) Proprietor: Telefield Medical Imaging Limited, Hong Kong (CN)
(72) Inventor: ZHENG, Yongping, Hong Kong (CN)
(74) Representative: Hellmich, Wolfgang
(86) International application number: PCT/CN2016/078835
(87) International publication number: WO 2017/016239

(56) References cited:
- CN-A- 101 822 549
- CN-A- 102 068 275
- CN-A- 102 626 324
- CN-A- 102 793 562
- CN-A- 103 142 246
- CN-A- 103 954 966
- JP-A- 2008 307 087
- US-A1- 2002 099 290
- US-A1- 2002 188 198
- US-A1- 2006 036 176
- US-A1- 2006 173 304
- US-A1- 2009 024 034
- US-A1- 2009 156 940
- US-A1- 2012 089 021
- US-A1- 2014 364 736

## Description

### TECHNICAL FIELD

The present application relates to the technical field of the ultrasonic scanning used in ultrasonic diagnostic instruments, and more particularly relates to a three-dimensional imaging ultrasonic scanning method.

### BACKGROUND

At present, in the ultrasonic diagnostic instruments, the three-dimensional ultrasound imaging can be accomplished by moving the B-ultrasound probe to a series of spatial positions for recording the B-ultrasound images (2D) at these spatial positions and reconstructing the three-dimensional ultrasound images based on the simultaneously recorded B-ultrasound images (2D). However, in the existing technology just a single one B-ultrasound probe is moved to do this, that is, the resulting ultrasound image is completely determined by the characteristics of the single one B-ultrasound probe, among them, which characteristics include ultrasound frequency, resolution, penetration depth, image width, image shape, focus mode, and image direction. However, in the three-dimensional ultrasound scanning, different organizations, different parts, different patients, different requirements, often have different requirements for these parameters, and meanwhile different requirements may need to be satisfied. For example, in the three-dimensional ultrasound imaging of the spine, an ultrasound probe with a higher frequency should be employed for the muscle tissue which may need high resolution, while an ultrasound probe with a lower frequency should also be employed for the deep tissue imaging. In this case, using a single one ultrasonic probe for the three-dimensional ultrasound imaging cannot meet the actual requirements.

US 2012/0089021 A1 refers to an ultrasonic scanning apparatus that includes a unique ultrasonic array to transmit ultrasonic energy to a biological structure in which the scanning data obtained by the imaging and therapeutic probes and video recording, if a video camera is employed is transmitted to the processor, processed to generate 3-D images of the treatment area. Information relating to the structure to be treated, such as the size, location, etc. of a lesion, is also inputted into the apparatus.

CN 102068275 A refers to an imaging system generates an image from the scan data, in which beam formation, detection, scan conversion, and/or rendering are used to generate each image.

CN 101822549A has disclosed an excitation and three-dimensional sensing integrated device for diagnosing breast cancer or craniocerebral injury, which comprises an acoustic unit and a thermoacoustic excitation unit, wherein the acoustic unit comprises a circular gear, one or more than one cambered ultrasonic array, a bowl-shaped cambered shell in which ultrasonic coupling liquid is filled and a protecting film. This excitation and three-dimensional sensing integrated device integrates the excitation and the sensing of three-dimensional thermoacoustic imaging, and after the integrated device is connected with necessary external equipment, three-dimensional thermoacoustic imaging, ultrasonic single imaging or ultrasonic joint imaging can be realized.

US 2002/0099290 A1 discloses a system and method for doing both transmission mode and reflection mode three-dimensional ultrasonic imagining, wherein such system comprises a first imaging head having a first array of transducer elements; a second imaging head having a second array of transducer elements; wherein said first and second arrays are arranged in a substantially two-dimensional pattern. The first and second arrays of the first and second imaging heads are each substantially interleaved with transmitting and receiving transducer elements. The first and second imaging heads are each adapted to rotate in the range of 0° to 360° around a target chamber to obtain a section of a full image scan. The first and second imaging heads are further adapted to translate in a direction substantially orthogonal to the rotation direction to substantially complete said full image scan.

US 2002/0188198 A1 has further disclosed a wearable breast tissue examination device including a support element adapted to fit over at least a portion of a breast of the wearer; wherein the support element has a shell, a measurement apparatus including at least two mutually opposed ultrasound transducer arrays disposed on at least a portion of the inner surface of the shell and at least one bladder element disposed in the shell that is configured to orient the wearer's breast properly for examination. The wearable device may also include means for operatively connecting the two mutually opposed transducer arrays to a transducer driver, and means for holding the support element on the wearer during use.

US 2006/0173304 A1 has disclosed apparatus and related methods are described for obtaining volumetric ultrasound scans of a breast of a supine patient in which a fluid reservoir including a bottom flexible membrane contacts an upward-facing surface of the breast. The fluid reservoir is filled with water or other suitable acoustically conductive fluid until the bottom membrane covering the breast is submerged. A transducer surface of an ultrasound probe is submerged in the fluid and moved over and/or around the breast area to obtain the ultrasound scans. Patient comfort is promoted, the patient being able to relax in a supine position during the procedure with a substantially uncompressed breast, imaging near the chest wall is enhanced, especially for patients having smaller-sized breasts, and also described are preferred embodiments using multiple transducer surfaces in which shadowing effects are reduced.

US 2009/0024034 A1 has disclosed using a conformal array or an ultrasound probe sequentially positioned at different locations, on scans of overlapping regions. A fiber optic sensor or light intensity based sensor determines the relative position of the probe or arrays for each of the scans. This relative position simplifies data correlation by limiting the search and/or is used to determine the relative position of the scans or data.

US 2014/0364739 A1 has disclosed synthetic-aperture ultrasound tomography systems and methods using scanning arrays and algorithms configured to simultaneously acquire ultrasound transmission and reflection data, and process the data for improved ultrasound tomography imaging, wherein the tomography imaging comprises total-variation regularization, or a modified total variation regularization, particularly with edge-guided or spatially variant regularization.

### SUMMARY

The object of the present application is to provide a three-dimensional imaging ultrasonic scanning method, aiming at the above defects of the prior art that a single one ultrasonic probe cannot satisfy the different requirements of the three-dimensional imaging in the three-dimensional ultrasonic scanning at the same time.

This object is achieved by the subject matter of the independent claim.

Preferred embodiments are the subject matter of the dependent claim.

The three-dimensional imaging ultrasound scanning method of an embodiment of the present invention employs at least two ultrasound B-ultrasound arrays having different parameters so that with just one time of scanning, a series of B-mode ultrasound images corresponding to each of the ultrasonic arrays are obtained. According to the actual needs, the series of images meeting the requirements are selected for the image three-dimensional ultrasound imaging. Two ultrasound probes with different shapes are used. Different ultrasonic arrays are installed in different directions so that three-dimensional images of multiple interested regions or three-dimensional images of the same interested region in different directions, such as the images of the spinous processes in different directions, are obtained by one time of three-dimensional scanning. In addition to satisfying different requirements by selecting images from different series of ultrasound images obtained by the different ultrasonic arrays, the corresponding images in different series are used for image fusion processing to achieve higher image quality, for example, the high signal to noise ratio of the image, thus providing a good foundation for the ultrasound diagnosis.

### BRIEF DESCRIPTION OF THE DRAWINGS

FIG. 1 is a flowchart of the three-dimensional imaging ultrasonic scanning method according to a first embodiment of the present application.
FIG. 2 is a schematic diagram of the structure of the motor driving device with a positioning function in FIG. 1, wherein the motor driving device is a circular scanning device.
FIG. 3 is a flowchart of the three-dimensional imaging ultrasonic scanning method according to a second embodiment of the present application.
FIG. 4 is an external view of a preferred embodiment of the first ultrasonic array and the second ultrasonic array in FIG. 3.
FIG. 5 is an external view of another preferred embodiment of the first ultrasonic array and the second ultrasonic array in FIG. 3.

### DETAILED DESCRIPTION OF THE PREFERRED EMBODIMENT

The present application provides a three-dimensional imaging ultrasonic scanning method applicable to the ultrasonic diagnostic instruments. The three-dimensional imaging ultrasonic scanning method can simultaneously satisfy different requirements for images during three-dimensional ultrasonic scanning. A specific solution is to simultaneously move at least two ultrasound B-ultrasound arrays with different parameters in a three-dimensional imaging scanning, so that a series of B-ultrasound images corresponding to each ultrasound array are obtained in a single one time of scanning. At the same time, combining with the spatial locator, the series of images meeting the requirements are selected for the image three-dimensional ultrasound imaging. In addition to satisfying different requirements by selecting images from different series of ultrasound images obtained by the different ultrasonic arrays, the corresponding images in different series are used for image fusion processing to achieve higher image quality, for example, the high signal to noise ratio of the image, thus providing a good foundation for ultrasonic diagnosis.

To make the object, the technical solution, and the advantage of the present application more clearly, the present application is further described in detail below with reference to the accompanying drawings and embodiments. It should be understood that the specific embodiments described herein are merely used to explain the present invention and are not intended to the present application.

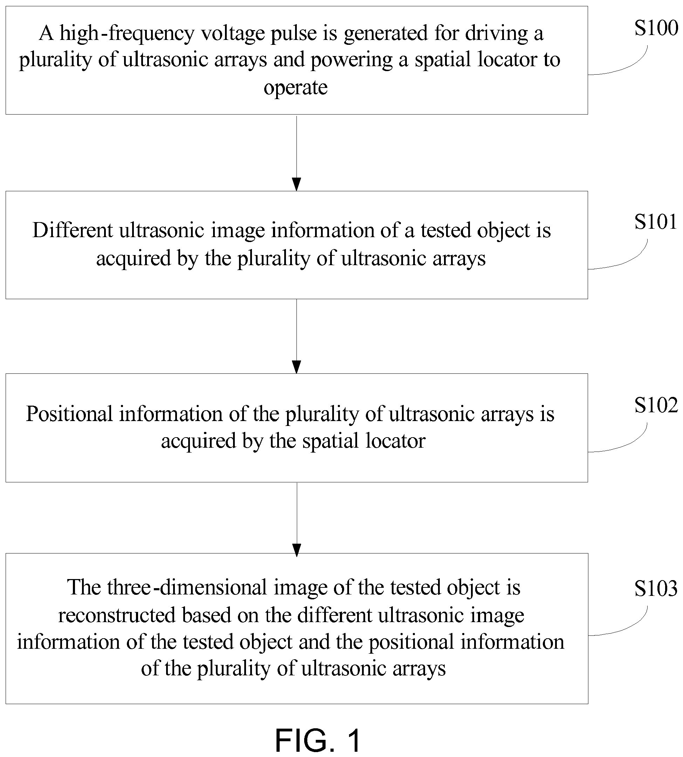

As shown in FIG.1, a flowchart of the three-dimensional imaging ultrasonic scanning method according to a first embodiment of the present application is disclosed. The three-dimensional imaging ultrasonic scanning method comprises the following steps.

In step S100, a high-frequency voltage pulse is generated for driving a plurality of ultrasonic arrays and powering a spatial locator to operate.

In this step, the high-frequency voltage pulse is generated by a transmission circuit which is positioned in the ultrasonic diagnostic instrument.

In this embodiment, the transmission circuit is composed of a clock generator, a frequency divider, a transmission delay circuit, and a pulse generator. The clock pulse generated by the clock generator is passed through the frequency divider to be lowered to a rate pulse with a certain frequency which is then passed through the transmission delay circuit to the pulse generator for generating a high frequency voltage pulse to drive the plurality of ultrasound arrays. That is, the transmission circuit transmits the electric signals to the plurality of ultrasonic arrays and drives the plurality of ultrasonic arrays, so that the plurality of ultrasonic arrays transmit the ultrasonic beams to the tested object, which belongs to the prior art and is not described herein again.

In step S101, different ultrasonic image information of a tested object is acquired by the plurality of ultrasonic arrays.

In this step, the plurality of ultrasonic arrays implement a scanning at the same time, or at different times or at fixed relative positions. During the scanning, the plurality of ultrasonic arrays respectively send ultrasonic waves to the tested object, receive the ultrasonic echo, and output corresponding electric signals according to the ultrasonic echo. Among them, the plurality of ultrasonic arrays have different frequencies, sizes, focus modes, shapes, mounting orientations, and the combinations thereof.

The above electrical signal is also needed to go through the amplifying circuit, the delay circuit and the addition circuit for further processing, so that the main part of the ultrasonic diagnostic instrument can better receive the electrical signal that represents the different information of the tested object. Wherein, the amplifying circuit is configured to perform low-noise amplification or buffering operation on the received or transmitted ultrasonic signals to better transmit the ultrasonic signals. The delay circuit and the addition circuit are respectively used to delay and add the electric signal of the ultrasonic wave.

In this embodiment, the ultrasonic arrays are arranged in different shapes to acquire images of different scanning ranges. The shapes of the ultrasonic arrays include a line array, an arc-shaped array, and a two-dimensional array. Wherein, an image of higher resolution is obtained when using the linear array for scanning, while an image of larger scanning range is obtained when using the arc-shaped array for scanning.

The mounting orientations of the plurality of ultrasonic arrays are different, images of multiple desired scanning areas or images of the same scanning area in different directions are obtained by one time of scanning.

In step S102, positional information of the plurality of ultrasonic arrays is acquired by the spatial locator.

In this step, the positional information of the plurality of ultrasonic arrays during the scanning is acquired by the spatial locator. During the scanning, the electric signals outputted by the ultrasonic arrays and the corresponding positional information are outputted to the main part of the ultrasonic diagnostic instrument. According to the scanning process, the spatial locator locates the positional information of the plurality of ultrasonic arrays to transmit the positional information to the main part of the ultrasonic diagnostic instrument for image-related processing.

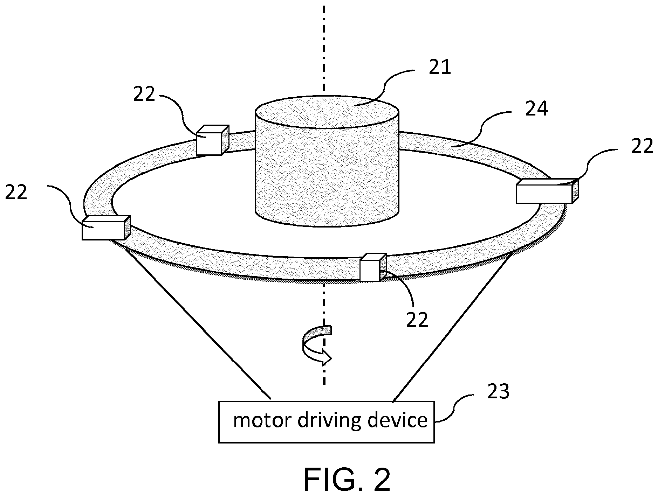

The spatial locator is a motor driving device with a positioning function. When the spatial locator is the motor driving device with a positioning function, the plurality of ultrasonic arrays are mounted at corresponding positions according to different forms of the motor driving device with a positioning function. When the motor driving device with a positioning function is a linear scanning device, the plurality of ultrasonic arrays are mounted on the linear scanning device. Such an embodiment does not fall within the scope of the invention. According to the invention, the motor driving device with a positioning function is a circular scanning device, the plurality of ultrasonic arrays are mounted on the circular scanning device. As shown in FIG.2, the circular scanning device includes a motor driver 23 and a supporting body 24 driven by the motor driver 23 to rotate. The supporting body 24 has a circular shape. The tested object 21 is placed at the center of the supporting body 24. The plurality of ultrasonic arrays 22 are mounted on the supporting body 24 and equally spaced along the circumference of the supporting body 24.

In step S103, the three-dimensional image of the tested object is reconstructed based on the different ultrasonic image information of the tested object and the positional information of the plurality of ultrasonic arrays.

In this step, the main part of the ultrasonic vibration apparatus acquires a scanned three-dimensional image based on the positional information acquired by the spatial locator and the electric signal of the different ultrasonic image information outputted by the ultrasonic arrays after image processing.

Specifically, a plurality of three-dimensional images of the tested object are reconstructed by performing image processing on the different ultrasonic image information of the tested object and the positional information of the plurality of ultrasonic arrays, wherein each three-dimensional image is reconstructed from the ultrasound image information obtained from each ultrasound array.

Alternatively, the three-dimensional image of the tested object is reconstructed by performing comprehensive image processing on the different ultrasonic image information of the tested object and the positional information of the plurality of ultrasonic arrays. Wherein, the three-dimensional image is obtained by reconstructing and fusing the different ultrasonic image information obtained by the plurality of ultrasonic arrays.

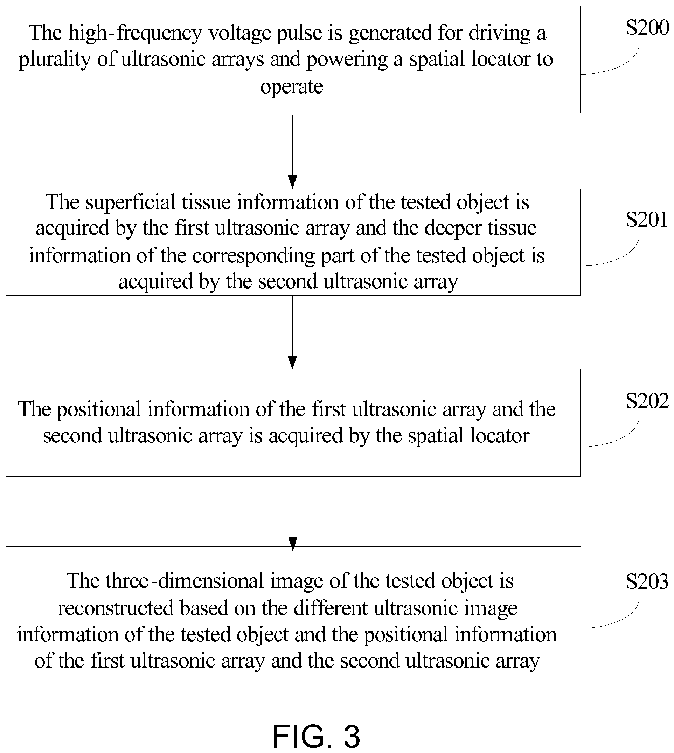

As shown in FIG.3, a flowchart of the three-dimensional imaging ultrasonic scanning method according to a second embodiment of the present application is disclosed, in which the plurality of ultrasonic arrays include a first ultrasonic array and a second ultrasonic array. Such an embodiment does not fall within the scope of the invention.

In this embodiment, the method comprises the following steps.

In step S200, the high-frequency voltage pulse is generated for driving a plurality of ultrasonic arrays and powering a spatial locator to operate.

In this step, the high-frequency voltage pulse is generated by a transmission circuit which is positioned in the ultrasonic diagnostic instrument.

In this embodiment, the transmission circuit can be composed of a clock generator, a frequency divider, a transmission delay circuit, and a pulse generator. The clock pulse generated by the clock generator is passed through the frequency divider to be lowered to a rate pulse with a certain frequency which is then passed through the transmission delay circuit to the pulse generator for generating a high frequency voltage pulse to drive the plurality of ultrasound arrays. That is, the transmission circuit transmits the electric signals to the plurality of ultrasonic arrays and drives the plurality of ultrasonic arrays, so that the plurality of ultrasonic arrays transmit the ultrasonic beams to the tested object, which belongs to the prior art and is not described herein again.

In step S201, the superficial tissue information of the tested object is acquired by the first ultrasonic array and the deeper tissue information of the corresponding part of the tested object is acquired by the second ultrasonic array.

In this step, the first ultrasonic array and the second ultrasonic array may implement a scanning at the same time, or at different times or at fixed relative positions. During the scanning, the first ultrasonic array and the second ultrasonic array respectively send ultrasonic waves to the tested object, receive the ultrasonic echo, and output corresponding electric signals according to the ultrasonic echo. Wherein, both the first ultrasonic array and the second ultrasonic array are linear arrays having the same mounting orientation but different frequencies.

In step S202, the positional information of the first ultrasonic array and the second ultrasonic array is acquired by the spatial locator.

In this step, the positional information of the first ultrasonic array and the second ultrasonic array during the scanning is acquired by the spatial locator. During the scanning, the electric signals outputted by the ultrasonic arrays and the corresponding positional information are outputted to the main part of the ultrasonic diagnostic instrument. According to the scanning process, the spatial locator locates the positional information of the plurality of ultrasonic arrays to transmit the positional information to the main part of the ultrasonic diagnostic instrument for image-related processing.

The spatial locator is the motor driving device with a positioning function, the first ultrasonic array and the second ultrasonic array are mounted at corresponding positions according to different forms of the motor driving device with a positioning function. When the motor driving device with a positioning function is a linear scanning device, the first ultrasonic array and the second ultrasonic arrays are mounted on the linear scanning device. When the motor driving device with a positioning function is a circular scanning device, the first ultrasonic array and the second ultrasonic array are mounted on the circular scanning device.

In step S203, the three-dimensional image of the tested object is reconstructed based on the different ultrasonic image information of the tested object and the positional information of the first ultrasonic array and the second ultrasonic array.

In this step, the main part of the ultrasonic vibration apparatus acquires a scanned three-dimensional image based on the positional information acquired by the spatial locator and the electric signals for the same part of the tested object at the different depths outputted by the first ultrasonic array and the second ultrasonic array after image processing.

Specifically, two three-dimensional images of the tested object are reconstructed by performing image processing on the different ultrasonic image information of the tested object and the positional information of the first ultrasonic array and the second ultrasonic array, wherein one three-dimensional image is reconstructed from the ultrasound image information obtained from the first ultrasound array, and the other three-dimensional image is reconstructed from the ultrasound image information obtained from the second ultrasound array.

Alternatively, the three-dimensional image of the tested object is reconstructed by performing comprehensive image processing on the different ultrasonic image information of the tested object and the positional information of the first ultrasonic array and the second ultrasonic array. Wherein, the three-dimensional image is obtained by reconstructing and fusing different ultrasonic image information obtained by the first ultrasonic array and the second ultrasonic array.

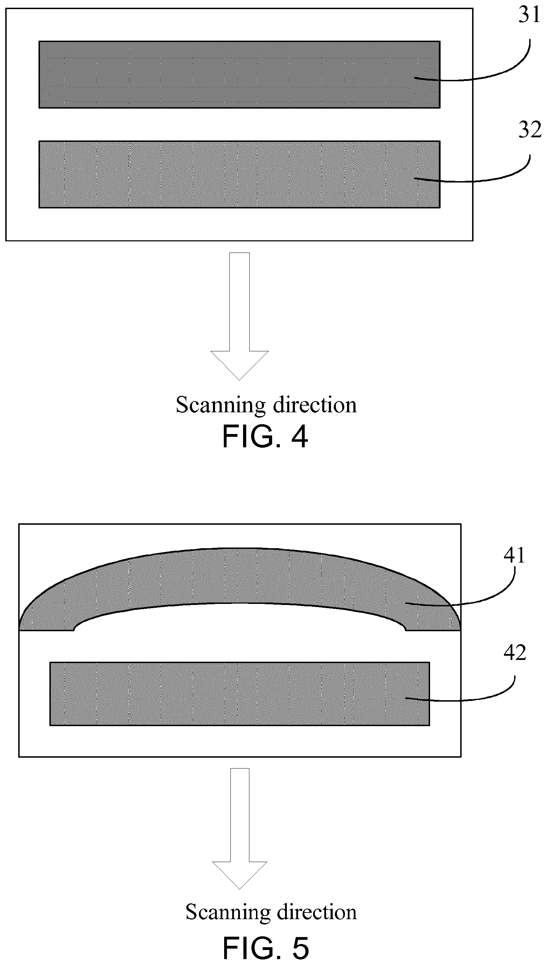

As shown in FIG.4, an external view of a preferred embodiment of the first ultrasonic array and the second ultrasonic array in FIG. 3 is disclosed. In this embodiment, both the first ultrasonic array 31 and the second ultrasonic array 32 are linear arrays arranged in parallel, that is, the mounting orientations of the first ultrasonic array 31 and the second ultrasonic array 32 are the same. During the scanning, the areas scanned by the first ultrasonic array 31 and the second ultrasonic array 32 are the same. The frequency of the first ultrasonic array 31 is *f*₀, and the frequency of the second ultrasonic array 32 is *f*₁. When *f*₀ and *f*₁ are not equal, the images of the same tested object at the different depths are obtained according to the different frequencies. In the present application, the mounting orientations of the first ultrasonic array 31 and the second ultrasonic array 32 are not limited to this. Meanwhile, other parameters of the ultrasonic arrays, that is, the shape and the size, are different according to practical needs.

As shown in FIG.5, an external view of another preferred embodiment of the first ultrasonic array and the second ultrasonic array in FIG. 3 is disclosed. In this embodiment, the first ultrasonic array 41 is arranged as a linear array, while the second ultrasonic array 42 is arranged as an arc-shaped array. The mounting orientations of the first ultrasonic array 41 and the second ultrasonic array 42 are the same. During scanning, a higher resolution is obtained by the scanning of the first ultrasonic array 41, and a larger scanning area is obtained by the scanning of the second ultrasonic array 42. In the present application, the mounting orientations of the first ultrasonic array 41 and the second ultrasonic array 42 are not limited to this. Meanwhile, other parameters of the ultrasonic arrays, that is, the shape and the size, are different according to practical needs.

In summary, the present application provides a three-dimensional imaging ultrasonic scanning method applicable to ultrasonic diagnostic instruments. The three-dimensional imaging ultrasonic scanning method can simultaneously satisfy different requirements for images during three-dimensional ultrasonic scanning. A specific solution is to simultaneously move at least two ultrasound B-ultrasound arrays with different parameters in a three-dimensional imaging scanning. Combining with the spatial locator, a series of B-ultrasound images corresponding to each ultrasound array are obtained in a single one time of scanning, so that the main part of the ultrasound diagnosis constructs a three-dimensional image of the tested object, thus providing a good foundation for the ultrasound diagnosis.

## Claims

1. A three-dimensional imaging ultrasonic scanning method applicable to ultrasonic diagnostic instruments, including following steps:
generating (S100) a high-frequency voltage pulse for driving a plurality of ultrasonic arrays and powering a spatial locator to operate;
acquiring (S101) different ultrasonic image information of a tested object (21) by the plurality of ultrasonic arrays (22), wherein the plurality of ultrasonic arrays respectively send ultrasonic waves to the tested object, receive ultrasonic echo from the tested object and output corresponding electrical signals according to the ultrasonic echo and further wherein the plurality of ultrasonic arrays have different size, focus modes, shapes and the combinations thereof;
further wherein the mounting orientations of the plurality of ultrasonic arrays are different and images of multiple desired scanning areas or images of the same scanning area in different directions are obtained by one time of scanning;
acquiring (S102) positional information of the plurality of ultrasonic arrays by the spatial locator;
the spatial locator is a motor driving device with a positioning function, the motor driving device with the positioning function is a circular scanning device; the circular scanning device includes a motor driver (23) and a supporting body (24) driven by the motor driver (23) to rotate; the supporting body (24) has a circular shape; the tested object (21) is placed at the center of the supporting body (24); the plurality of ultrasonic arrays are mounted on the supporting body (24) and equally spaced along the circumference of the supporting body (24);
and the three-dimensional imaging ultrasonic scanning method further comprises:
reconstructing (S103) a three-dimensional image of the tested object (21) based on the different ultrasonic image information of the tested object (21) and the positional information of the plurality of ultrasonic arrays; wherein said positional information is acquired by the spatial locator and said ultrasonic image information is acquired from the electric signals outputted by the plurality of ultrasonic arrays after image processing.

2. The three-dimensional imaging ultrasonic scanning method according to claim 1, **characterized in that**, the plurality of ultrasonic arrays implement a scanning at the same time, or at different times or at fixed relative positions.

## Patentansprüche

1. Dreidimensionales bildgebendes Ultraschallabtastverfahren für Ultraschall-Diagnosegeräte, das die folgenden Schritte umfasst:
Erzeugen (S100) eines Hochfrequenz-Spannungsimpulses zum Ansteuern einer Mehrzahl von Ultraschallarrays und zum Betreiben eines räumlichen Lokalisierers;
Erfassen (S101) verschiedener Ultraschallbildinformationen eines untersuchten Objekts (21) durch die Mehrzahl von Ultraschallarrays (22), wobei die Mehrzahl von Ultraschallarrays jeweils Ultraschallwellen zu dem untersuchten Objekt senden, Ultraschallechos von dem untersuchten Objekt empfangen und entsprechende elektrische Signale gemäß dem Ultraschallecho ausgeben, und wobei ferner die Mehrzahl von Ultraschallarrays verschiedene Größen, Fokusmodi, Formen und Kombinationen davon aufweisen;
wobei weiter die Montageausrichtungen der mehreren Ultraschallarrays unterschiedlich sind und Bilder mehrerer gewünschter Abtastbereiche oder Bilder desselben Abtastbereichs in verschiedenen Richtungen durch einmaliges Abtasten erhalten werden;
Erfassen (S102) von Positionsinformationen von der Mehrzahl von Ultraschallarrays durch den räumlichen Lokalisierer;
wobei der räumliche Lokalisierer eine Motorantriebsvorrichtung mit einer Positionierungsfunktion ist, wobei die Motorantriebsvorrichtung mit der Positionierungsfunktion eine kreisförmige Abtastvorrichtung ist; wobei die kreisförmige Abtastvorrichtung einen Motortreiber (23) und einen Stützkörper (24) enthält, der durch den Motortreiber (23) angetrieben wird, um sich zu drehen; wobei der Stützkörper (24) eine kreisförmige Form hat; wobei das untersuchte Objekt (21) in der Mitte des Stützkörpers (24) angeordnet ist; wobei die Mehrzahl von Ultraschallarrays auf dem Stützkörper (24) angebracht und gleichmäßig entlang des Umfangs des Stützkörpers (24) beabstandet sind;
und wobei das dreidimensionale bildgebende Ultraschallabtastverfahren ferner:
das Rekonstruieren (S103) eines dreidimensionalen Bildes des untersuchten Objekts (21) auf der Grundlage der verschiedenen Ultraschallbildinformationen des untersuchten Objekts (21) und der Positionsinformationen der mehreren Ultraschallarrays umfasst; wobei die Positionsinformationen durch den räumlichen Lokalisierer erfasst werden und die Ultraschallbildinformationen aus den elektrischen Signalen erfasst werden, die von den mehreren Ultraschallarrays nach der Bildverarbeitung ausgegeben werden.

2. Dreidimensionales bildgebendes Ultraschallabtastverfahren gemäß Anspruch 1, wobei die mehreren Ultraschallarrays eine Abtastung zur gleichen Zeit oder zu unterschiedlichen Zeiten oder an festen relativen Positionen durchführen.

## Revendications

1. Procédé de balayage ultrasonore d'imagerie tridimensionnelle applicable à des instruments de diagnostic ultrasonore, comprenant les étapes suivantes :
la génération (S100) d'une impulsion de tension à haute fréquence pour entraîner une pluralité de batteries ultrasonores et alimenter un système de positionnement dans l'espace à actionner ;
l'acquisition (S101) de différentes informations d'image ultrasonores d'un objet testé (21) par la pluralité de batteries ultrasonores (22), dans lequel la pluralité de batteries ultrasonores envoient respectivement des ondes ultrasonores à l'objet testé, reçoivent un écho ultrasonore provenant de l'objet testé et émettent des signaux électriques correspondants en fonction de l'écho ultrasonore et dans lequel en outre la pluralité de batteries ultrasonores ont différentes tailles, modes de focalisation et formes et combinaisons de ceux-ci ;
dans lequel en outre les orientations de fixation de la pluralité de batteries ultrasonores sont différentes et les images de multiples zones ou images de balayage souhaitées de la même zone de balayage sont obtenues dans différentes directions par un balayage dans le temps ;
l'acquisition (S102) des informations de position de la pluralité de batteries ultrasonores par le système de positionnement dans l'espace ;
le système de positionnement dans l'espace est un dispositif d'entraînement de moteur avec une fonction de positionnement, le dispositif d'entraînement de moteur avec la fonction de positionnement est un dispositif de balayage circulaire ;
le dispositif de balayage circulaire comprend un dispositif d'entraînement de moteur (23) et un corps de maintien (24) entraîné en rotation par le dispositif d'entraînement de moteur (23) ; le corps de maintien (24) a une forme circulaire ; l'objet testé (21) est placé au centre du corps de maintien (24) ; la pluralité de batteries ultrasonores sont fixées sur le corps de maintien (24) et sont équidistantes le long de la circonférence du corps de maintien (24) ;
et le procédé de balayage ultrasonore d'imagerie tridimensionnelle comprend en outre :
la reconstruction (S103) d'une image tridimensionnelle de l'objet testé (21) sur la base des différentes informations d'image ultrasonores de l'objet testé (21) et les informations de positionnement de la pluralité de batteries ultrasonores ; dans lequel lesdites informations de position sont acquises par le système de positionnement dans l'espace et lesdites informations d'image ultrasonores sont acquises à partir des signaux électriques émis par la pluralité de batteries ultrasonores après le traitement d'image.

2. Procédé de balayage ultrasonore d'imagerie tridimensionnelle selon la revendication 1, **caractérisé en ce que** la pluralité de batteries ultrasonores réalisent un balayage au même moment ou à différents moments ou à des positions relatives fixes.
